(19) Europäisches Patentamt
European Patent Office
Office européen des brevets

(11) Veröffentlichungsnummer : **0 336 215 B1**

# (12) EUROPÄISCHE PATENTSCHRIFT

(45) Veröffentlichungstag der Patentschrift : **15.05.91 Patentblatt 91/20**

(51) Int. Cl.$^5$ : **B01J 31/16,** C07C 51/56, C07C 53/12, B01J 31/18

(21) Anmeldenummer : **89105111.2**

(22) Anmeldetag : **22.03.89**

## (54) Trägerkatalysator für die Herstellung von Monocarbonsäureanhydriden.

(30) Priorität : **02.04.88 DE 3811344**

(43) Veröffentlichungstag der Anmeldung : **11.10.89 Patentblatt 89/41**

(45) Bekanntmachung des Hinweises auf die Patenterteilung : **15.05.91 Patentblatt 91/20**

(84) Benannte Vertragsstaaten : **AT BE CH DE FR GB IT LI NL**

(56) Entgegenhaltungen :
**EP-A- 0 079 461**
**EP-A- 0 180 802**

(56) Entgegenhaltungen :
**EP-A- 0 185 882**
**EP-A- 0 245 893**
**EP-A- 0 263 564**
**US-A- 4 452 119**

(73) Patentinhaber : **HOECHST AKTIENGESELLSCHAFT**
**Postfach 80 03 20**
**W-6230 Frankfurt am Main 80 (DE)**

(72) Erfinder : **Luft, Gerhard, Prof. Dr.**
**Ludwigstrasse 141a**
**W-6109 Mühltal (DE)**
Erfinder : **Trabold, Peter, Dr.**
**Ahornweg 19a**
**W-6110 Dieburg (DE)**

EP 0 336 215 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft einen Trägerkatalysator für die Herstellung von Monocarbonsäureanhydriden durch Carbonylierung der entsprechenden Ester oder Ether, worin das Trägermaterial eine Edelmetall-Chelatverbindung trägt, die aus einer Edelmetallverbindung der 8. Nebengruppe des Periodensystems der Elemente und einem Chelator mit Organostickstoff-, Organophosphor-, Organoarsen-, Organoschwefel- oder Mercaptogruppen gebildet ist.

Ein solcher Trägerkatalysator ist aus der DE-Offenlegungsschrift 3 511 048 bekannt.

Der erfindungsgemäße Trägerkatalysator ist insbesondere bestimmt für die Herstellung von Monocarbonsäureanhydriden der allgemeinen Formel $(RCO)_2O$ durch Umsetzung eines Carbonsäureesters oder Dialkylethers der allgemeinen Formel RCOOR bzw. ROR, wobei R jeweils denselben Alkylrest mit 1 bis 4 C-Atomen bedeutet, mit Kohlenmonoxid in der Gasphase in Gegenwart von Jod oder Brom oder deren Verbindungen als Reaktionspromotor bei Temperaturen von 130 bis 400°C und Drücken von 1 bis 150 bar.

Ein derartiges, in der Gasphase mit einem Trägerkatalysator arbeitendes Verfahren ist bereits aus der DE-Offenlegungsschrift 3 511 050 bekannt, welche die bei den Flüssigphaseverfahren auftretenden Nachteile, z.B. die schwierige Abtrennung und Rückführung von suspendiertem und teilweise gelöstem Katalysator und ggf. Promotor, vermeidet.

Es ist Aufgabe der vorliegenden Erfindung, den Chelator so zu modifizieren, daß sich Standzeit (Aktivitätsdauer) und Selektivität des Trägerkatalysators bei gleichem Trägermaterial deutlich verbessern.

Im einzelnen ist der Trägerkatalysator der Erfindung dadurch gekennzeichnet, daß

1. der Chelator in seinem Grundkörper mit Alkyl-, Aryl- oder Aralkylgruppen substituiert ist und eine dere folgenden Strukturformeln aufweist:

a) 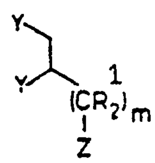

b) 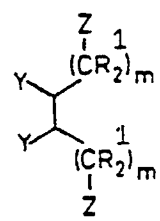

wobei
Y = $-NR^2_2$, ein stickstoffhaltiger Arylrest, $-AsR^2_2$, $-PR^2_2$, $-SR^2$ oder –SH ;
Z = –H, Aryl, Phenyl (ggf. ortho-, meta- oder parasubstituiert)
$R^1$ = –H, $C_1$ bis $C_3$-Alkyl ;
$R^2$ = $C_1$ bis $C_6$-Alkyl, $C_5$ oder $C_6$-Cycloalkyl, $-C_6H_5$ oder $C_6H_5CH_2-$, die ggf. mit Halogen-, Methoxy-, Ethoxy- oder $C_1$ bis $C_3$-Alkylgruppen substituiert sind ;
m = 2-6, vorzugsweise 2-4.

Der Trägerkatalysator der Erfindung kann weiterhin wahlweise und bevorzugt dadurch gekennzeichnet sein, daß

2. in ihm das Trägermaterial zusätzlich eine Nichtedelmetall-Chelatverbindung trägt, die aus einer Nichtedelmetallverbindung aus der 6. oder 8. Nebengruppe des Periodensystems der Elemente und einem Chelator nach Punkt 1 gebildet ist.

3. er zusätzlich Nichtedelmetallverbindungen aus der 1. und 3. Hauptgruppe oder der 4. und 6. oder der 8. Nebengruppe des Periodensystems der Elemente als Promotoren enthält.

4. er ein anorganisches oxidisches Trägermaterial oder einen Aktivkohleträger enthält.

5. er zusammen 0.01 bis 50 Gew.%, vorzugsweise 0.1 bis 20 Gew.%, Chelatverbindung und ggf. Nichtedelmetallverbindungen enthält.

Als Katalysatorträger kommen bevorzugt anorganische Oxide wie z.B. $SiO_2$, $Al_2O_3$, MgO, $TiO_2$, $La_2O_3$, $ZrO_2$, Zeolith, Ton, NiO, $Cr_2O_3$, $WO_3$ oder entsprechende Mischoxide, aber auch Aktivkohle in Frage, welche BET-Oberflächen von 1 bis 1000 $m^2/g$, vorzugsweise 30 bis 400 $m^2/g$, haben.

Ebenso wie gemäß DE-Offenlegungsschrift 35 11 048 sind die Promotoren der 5. oder 6. Hauptgruppe in den erfindungsgemäß eingesetzten Chelatoren bzw. Chelatliganden chemisch gebunden. Sie bilden selbst eine funktionelle Gruppe, die die Edelmetallverbindungen aus der Gruppe VIII und ggf. Nichtedelmetallverbindungen aus der 6. oder 8. Nebengruppe chelatisieren. Es ist ein Vorteil, daß die zur Steigerung der Aktivität und Selektivität der Trägerkatalysatoren notwendigen Promotoren aus der 5. oder 6. Hauptgruppe des Periodensystems der Elemente eine funktionelle Gruppe Y in den Chelatoren bilden und somit fixiert sind, weil dadurch eine Abtrennung und Rückführung dieser z.B. Organostickstoff- oder Organophosphorpromotoren entfällt.

Das Verfahren zur Herstellung von Monocarbonsäureanhydriden, katalysiert mit dem Trägerkatalysator der Erfindung, weist gegenüber dem Verfahren der DE-OS 35 11 050 höhere Selektivitäten und besonders bei Langzeitbelastung höhere Standzeiten des Trägerkatalysators auf.

Ein weiterer Vorteil der Erfindung ist darin zu sehen, daß die auf dem Trägermaterial aufgetragenen modifizierten Edelmetall-Chelatverbindungen und ggf. Nichtedelmetall-Chelatverbindungen noch höhere Schmelzpunkte (240-270°C) als die in der DE-OS 35 11 048 beschriebenen Komplexe besitzen, was zu einer höheren thermischen Stabilität der Katalysatoren bzw. zu einer Erhöhung des Anwendungsbereichs von 20 bis 50°C führt.

Der Trägerkatalysator der Erfindung dient insbesondere zur Herstellung von Essigsäureanhydrid aus Methylacetat oder Dimethylether in Gegenwart von Methyljodid oder Methylbromid als Reaktionspromotor. Als Reaktionspromotor kann auch HI, HBr oder allgemein RI oder RBr eingesetzt werden, wobei R einen Alkylrest mit 1 bis 4 C-Atomen darstellt.

Die Trägermaterialien wurden bereits genannt; als Mischoxide kommen z.B. $Cr_2O_3$-$Al_2O_3$, $WO_3$-$Al_2O_3$, $MgO$-$Al_2O_3$, $SiO_2$-$Al_2O_3$ oder $ZrO_2$-$Al_2O_3$ in Frage. Der Trägerkatalysator enthält bevorzugt 0.05 bis 5 Gew.% Edelmetall und wird in einer Korngröße von 1 bis 20 mm eingesetzt.

Als Edelmetallverbindungen können bei der Herstellung des Trägerkatalysators z.B. folgende Verbindungen eingesetzt werden :

Rhodium :

$RhCl_3$, $RhCl_3 \cdot 3\ H_2O$, $RhBr_3$, $RhI_3$, $Rh(NO_3)_3$, $Rh_2(CO)_4Cl_2$, $Rh_2(CO)_4Br_2$, $Rh(CO)_4I_2$, $[P(C_6H_5)_3]_3RhCl$, $[P(C_6H_5)_3]_2Rh(CO)Cl$, $Rh_6(CO)_{16}$, $Rh_4(CO)_{12}$, $Rh_2(O_2CCH_3)_4$, $[RhCl(C_8H_{12})]_2$ ;

Iridium :

$IrCl_3$, $[Ir(CO)_3Cl]_2$, $Ir[P(C_6H_5)_3]_2(CO)Cl$, $Ir_4(CO)_{12}$, $[IrCl(C_8H_{12})]_2$, $Cl(CO)_2Irpyr$ (pyr = $C_6H_5N$) ;

Palladium :

$PdCl_2$, $PdBr_2$, $PdI_2$, $(CH_3CO_2)_2Pd[P(C_6H_5)_3]_2$, $PdCl_2[P(C_6H_5)_3]_2$, $Pd(O_2CCH_3)_2$, $PdCl_2(C_8H_{12})$, $(C_6H_5CN)_2PdCl_2$;

Ruthenium :

$RuCl_3$, $Ru_3(CO)_{12}$, $RuCl_2[P(C_6H_5)_3]_3$, $RuCl_2(CO)_2[P(C_6H_5)_3]_2$, $[RuCl_2(CO)_3]_2$.

Als Nichtedelmetallverbindungen aus der 6. oder 8. Nebengruppe, insbesondere Cr, Ni, aber auch W, Fe, Co, die ebenfalls mit den Chelatoren reagieren, kommen z.B. ferner in Frage :

Chrom :

$Cr(CO)_6$, $CrCl_3$, $C_7H_8Cr(CO)_3$.

Nickel :

$Ni(CO)_4$, $[P(C_6H_5)_3]_2Ni(CO)_2$, $NiCl_2$, $Ni(C_8H_{12})_2$.

Als Nichtedelmetallverbindungen aus der 1. bis 3. Hauptgruppe oder der 4. bis 6. oder der 8. Nebengruppe des Periodensystems, vorzugsweise des Li, Na, Mg, Ca, Al, Ti, Zr, V, Cr, W, Fe, Co, Ni, können z.B. Hydroxide, Carbonate, Carbonyle, Hydride, Halogenide und andere Salze eingesetzt werden. Diese Verbindungen unedler Metalle können zusätzlich, z.B. als Lösung durch Imprägnierung, auf den Katalysatorträger aufgebracht werden.

Zur Herstellung des erfindungsgemäßen Trägerkatalysators muß zuerst der Chelator mit den funktionellen Gruppen Y bereitgestellt werden. Dieser kann nach oder in Analogie zu Literaturangaben dargestellt werden. Im allgemeinen wird dann eine der genannten Edelmetallverbindungen aus der Gruppe VIII und ggf. eine der genannten Nichtedelmetallverbindungen aus der 6. oder 8. Nebengruppe in Lösung mit dem Chelator in Verbindung gebracht, wobei Chelatverbindungen entstehen, deren Schmelzpunkte über der Temperatur der Carbonylierungsreaktion zur Herstellung von Monocarbonsäureanhydriden liegen.

Anschließend erfolgt die Imprägnierung des Trägermaterials mit den gelösten Chelatverbindungen zum fertigen Trägerkatalysator. Die Lösemittel für die Chelatverbindungen können polar oder unpolar sein, z.B. Dichlormethan, Chloroform, Methanol, Benzol, Toluol oder Xylol, worin das Trägermaterial suspendiert wird. Alle weiteren Einzelheiten zu Synthesen ergeben sich aus der Beschreibung der Katalysatorherstellung.

Das Mengenverhältnis von Carbonsäureester bzw. Dialkylether und Jod(verbindung) oder Brom(verbindung) in der Reaktionszone kann innerhalb weiter Grenzen schwanken. Im allgemeinen beträgt die Menge des Carbonsäureesters und/oder Dialkylethers 1 bis 500 mol, vorzugsweise 1 bis 100 mol, je 1 mol Jod(verbindung) oder Brom(verbindung). Die Temperatur der Reaktionszone wird so gewählt, daß das Reaktionsgemisch bei jedem beliebigen Umsatz gasförmig vorliegt. Bevorzugt wird die Temperatur zwischen 150 und 250°C gewählt. Der bevorzugte Druck liegt zwischen 5 und 30 bar.

Die Verweilzeit des Reaktionsgemisches am festen Trägerkatalysator der Erfindung beträgt 1 bis 1000 s, bevorzugt 1 bis 180 s. Die Umsetzung kann in einem senkrecht angeordneten und mit Trägerkatalysator gefüllten Strömungsrohr oder auch in einem Rühr- oder Schüttelautoklaven erfolgen, der den Trägerkatalysator enthält.

Das aus der Carbonylierungszone abströmende Reaktionsgemisch ist gasförmig und enthält Kohlenmonoxid, Methyljodid, Essigsäureanhydrid, nichtumgesetztes Methylacetat oder Dimethylether und ggf. sehr geringe Mengen Essigsäure.
Das gasförmige Reaktionsgemisch wird abgekühlt, Essigsäureanhydrid und ggf. Essigsäure auskondensiert und die nicht kondensierten Stoffe wie CO, Methyljodid, Methylacetat oder Dimethylether in die Reaktionszone zurückgeführt. Die umgesetzten Anteile von Ester oder Ether sowie CO werden fortlaufend ersetzt.
Die einfache Abtrennung der Anhydride durch Kühlung des abströmenden Reaktionsgemisches und Rückführung der nicht kondensierbaren Gase stellt, wie beim Verfahren der DE-OS 35 11 050, einen wesentlichen Vorteil des mit dem erfindungsgemäßen Trägerkatalysator durchgeführten Verfahrens dar, da dies ohne komplizierte Trennoperationen erfolgen kann. Der erfindungsgemäße Trägerkatalysator wird nicht verunreinigt und verbleibt in der Reaktionszone, was den gesamten Verfahrensablauf bedeutend vereinfacht.

Beispiele

Beschreibung der Katalysatorherstellung

In allen Fällen wurden die Katalysatorträger zur Aktivierung zuvor bei 200°C und 0.1 mbar 10 h getrocknet. Nach Aufbringen der Metallkomponente erhitzte man die Katalysatoren 8 h mit Chlortrimethylsilan zum Sieden und trocknete anschließend bei 0.1 mbar und 100°C. Sämtliche Synthesen wurden in Argonatmosphäre unter Ausschluß von Luftsauerstoff und Wasser ausgeführt. Alle verwendeten Lösemittel wurden zuvor über Molekularsieb 4 A oder wenn möglich mit Benzophenonnatrium getrocknet.

Rührautoklavversuche

Man verwendet einen 0.25 Liter fassenden Rührautoklaven aus korrosionsfreiem Edelstahl (Hastelloy C) mit den erforderlichen Zu- und Ableitungen, der einen drehbaren Katalysatorkorb enthält.
Die Carbonsäureester oder Dialkylether werden gasförmig in Gegenwart des bewegten, festen Trägerkatalysators mit CO-Gas umgesetzt. Der Trägerkatalysator befindet sich in dem drehbaren Katalysatorkorb, der gleichzeitig für die Durchmischung der Gase sorgt.
Der Autoklav wird mit 2.5 ml eines flüssigen Gemisches aus 20 Volumenteilen Methyljodid und 80 Volumenteilen Ester oder Ether beschickt und auf Reaktionstemperatur aufgeheizt. Die Carbonylierung wird durch Aufpressen von Kohlenmonoxid eingeleitet. Der CO-Druck wird durch regelmäßiges Nachdrücken konstant gehalten.
Die Einzelheiten der Versuchsdurchführungen ergeben sich aus den Beispielen.

In den nachfolgenden Beispielen bedeutet Ø = $C_6H_5$–.

Beispiel 1

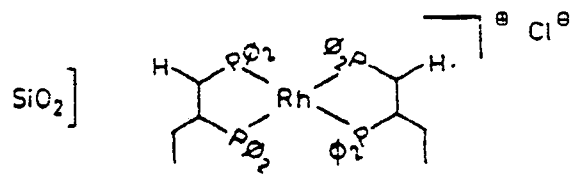

65.4 g aktivierte Siliciumdioxidpellets 3,175 mm x 3,175 mm (1/8" x 1/8") (95% $SiO_2$) mit einer inneren Oberfläche nach BET von 68 $m^2/g$ und einem Porenvolumen von 0.43 ml/g wurden mit 150 ml einer Lösung aus 629 mg des Komplexes 4 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charackterisierung : | Gelbe Pellets |
| Rh-Gehalt : | 0.1 Gew.% |

Syntheseweg des Rhodiumkomplexes 4

1,2-Dichlorbutan (2) :

2 kann durch Umsetzung von 1-Buten (1) mit Chlor bei 0°C in Dichlormethan in fast quantitativer Ausbeute erhalten werden.

1,2-Bis(diphenylphosphino)butan (3) :

3 wird durch Umsetzung doppeltmolarer Menge Natriumdiphenylphosphid in Dioxan mit 2, gelöst in Tetrahydrofuran, bei Raumtemperatur synthetisiert [analog 1,2-Bis(diphenylphosphino)ethan ; siehe K. Issleib und D.-W. Müller, Chem. Ber. 92, 3175 (1959)]. Ausbeute 78%.

[1,2-Bis(diphenylphosphino)butan]rhodium(I)-chlorid (4) :

Eine Lösung von 4 mmol 3 in Benzol wird unter Rühren zu einer Lösung von 1 mmol Dichlorotetracarbonyldirhodium in Benzol getropft, wobei der Komplex 4 analysenrein ausfällt. Ausbeute 94% Vgl. Synthese von [1,2-Bis(diphenylphosphino)ethan]rhodium(I)-chlorid, A. Sacco et al., J. Chem. Soc. (London), 3274 (1964).

2 ml (1.86 g) Essigsäuremethylester, 0.5 ml (1.14 g) Methyljodid und 6.46 g des Katalysators werden im Autoklaven mit Kohlenmonoxid bei 15 bar CO-Druck und 180°C umgesetzt. Nach 1 h Reaktionszeit ergibt sich eine Katalysatorleistung von 23 g $Ac_2O/g_{Rh} \cdot h$, bei einer Selektivität von 98%.

Ein Stahlrohr von 20 mm Durchmesser und 400 mm Länge wird als Strömungsrohr senkrecht angeordnet und mit 52.8 g des Katalysators gefüllt. Bei einem Druck von 12 bar und einer Temperatur von 180°C werden stündlich 8 Nl CO (Nl = Liter, gemessen bei 1.013 bar und 0°C) sowie ein verdampftes Gemisch (12.9 ml Flüssigkeit) aus Methyljodid und Methylacetat (Molverhältnis 1 : 4) durch das Strömungsrohr geleitet.

Das abströmende Reaktionsgemisch wird gaschromatographisch on-line analysiert. Hierbei ergibt sich eine Raum-Zeit-Ausbeute von 15.4 g $Ac_2O/g_{Rh} \cdot h$, bei einer Selektivität von 99%.

Unter diesen Reaktionsbedingungen wurde die Carbonylierung 280 Stunden durchgeführt, wobei der eingesetzte Trägerkatalysator keinen Aktivitätsverlust zeigte.

Beispiel 2

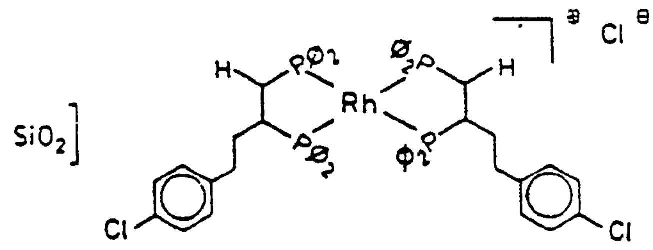

68.3 g aktivierte Siliciumdioxidpellets 3,175 mm × 3,175 mm (1/8" · 1/8") (95% $SiO_2$) mit einer inneren Oberfläche nach BET von 68 $m^2/g$ und einem Porenvolumen von 0.43 ml/g wurden mit 150 ml einer Lösung aus 723 mg des Komplexes 8 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charakterisierung : | Gelbe Pellets |
| Rh-Gehalt : | 0.09 Gew% |

Syntheseweg des Rhodiumkomplexes 8

1,2-Dichlor-4-(4-chlorphenyl)butan (6) :

6 kann durch Umsetzung von 4-(4-Chlorphenyl)buten (5) mit Chlor bei 0°C in Dichlormethan synthetisiert werden. Ausbeute 93%.

1,2-Bis(diphenylphosphino)-4-(4-chlorphenyl)-butan (7) :

7 wird durch Umsetzung doppeltmolarer Menge Natriumdiphenylphosphid in Dioxan mit 6, gelöst in Tetrahydrofuran, bei Raumtemperatur mit 82% Ausbeute synthetisiert [analog 1,2-Bis(diphenylphosphino)ethan ; siehe K. Issleib und D.-W. Müller, Chem. Ber. 92, 3175 (1959)].

[1,2-Bis(diphenylphosphino)-4-(4-chlorphenyl)butan]-rhodium(I)-chlorid (8) :

Eine Lösung von 4 mmol 7 in Benzol wird unter Rühren zu einer Lösung von 1 mmol Dichlorotetracarbonyldirhodium in Benzol getropft, wobei der Komplex 8 analysenrein ausfällt. Ausbeute 96%. Vgl. Synthese von [1,2-Bis(diphenylphosphino)ethan]rhodium(I)-chlorid ; A. Sacco et al., J. Chem. Soc. (London), 3274 (1964).

2 ml (1.86 g) Essigsäuremethylester, 0.5 ml (1.14 g) Methyljodid und 6.77 g des Katalysators werden im Autoklaven mit Kohlenmonoxid bei 15 bar CO-Druck und 180°C umgesetzt. Nach 1 h Reaktionszeit ergibt sich eine Katalysatorleistung von 22 g $Ac_2O/g_{Rh} \cdot h$, bei einer Selektivität von 98%.

Ein Stahlrohr von 20 mm Durchmesser und 400 mm Länge wird als Strömungsrohr senkrecht angeordnet und mit 51.0 g des Katalysators gefüllt. Bei einem Druck von 12 bar und einer Temperatur von 180°C werden stündlich 8 Nl CO (Nl = Liter, gemessen bei 1.013 bar und 0°C), sowie ein verdampftes Gemisch (13.5 ml Flüssigkeit) aus Methyljodid und Methylacetat (Molverhältnis 1 : 4) durch das Strömungsrohr geleitet.

Das abströmende Reaktionsgemisch wird gaschromatographisch on-line analysiert. Hierbei ergibt sich eine Raum-Zeit-Ausbeute von 14.3 g $Ac_2O/g_{Rh} \cdot h$, bei einer Selektivität von 99%.

Unter diesen Reaktionsbedingungen wurde die Carbonylierung 280 Stunden durchgeführt, wobei der eingesetzte Trägerkatalysator keinen Aktivitätsverlust zeigte.

Beispiel 3

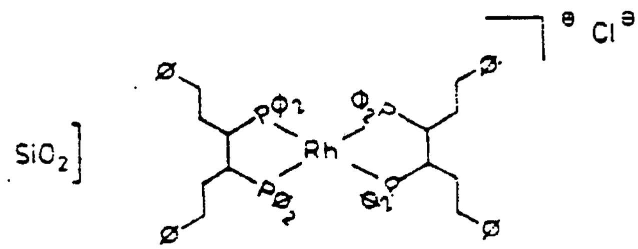

12.8 g aktivierte Siliciumdioxidpellets 3,175 mm × 3,175 mm (1/8". 1/8") (95% $SiO_2$) mit einer inneren Oberfläche nach BET von 68 $m^2/g$ und einem Porenvolumen von 0.43 ml/g wurden mit 50 ml einer Lösung aus 134.6 mg des Komplexes 12 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charakterisierung : | Gelbe Pellets |
| Rh-Gehalt : | 0.08 Gew.% |

Syntheseweg des Rhodiumkomplexes 12

1,6-Diphenylhex-3-en (9) :

Das Alken 9 wurde durch Umsetzung von Dihydrozimtaldehyd mit 3-Phenylpropyltriphenylphosphoniumbromid in 64%iger Ausbeute synthetisiert [Wittigreaktion : siehe Organikum, 15. Aufl., S. 494, VEB Deutscher Verlag der Wissenschaften, Berlin 1977].

3,4-Dichlor-1,6-diphenylhexan (10) :

10 kann durch Umsetzung von 9 mit Chlor bei 0°C in Dichlormethan synthetisiert werden. Ausbeute 96%.

3,4-Bis(diphenylphosphino)-1,6-diphenylhexan (11) :

11 wird durch Umsetzung doppeltmolarer Menge Natriumphenylphosphid in Dioxan mit 10, gelöst in Tetrahydrofuran, bei Raumtemperatur mit 74% Ausbeute synthetisiert [analog 1,2-Bis(diphenylphosphino)-ethan ; siehe K. Issleib und D.-W. Müller, Chem. Ber. 92, 3175 (1959)].

[3,4-Bis(diphenylphosphino)-1,6-diphenylhexan]-rhodium (I)-chlorid (12) :

Eine Lösung von 4 mmol 11 in Benzol wird unter Rühren zu einer Lösung von 1 mmol Dichlorotetracarbonyldirhodium in Benzol getropft, wobei der Komplex 12 ausfällt. Ausbeute 97%. Vgl. Synthese von [1,2-Bis(diphenylphosphino)ethan] rhodium(I)-chlorid ; A. Sacco et al., J. Chem. Soc. (London) 3274 (1964).

2 ml (1.86 g) Essigsäuremethylester, 0.5 ml (1.14 g) Methyljodid und 7.98 g des Katalysators werden im Autoklaven mit Kohlenmonoxid bei 15 bar CO-Druck und 180°C umgesetzt. Nach 1 h Reaktionszeit ergibt sich eine Katalysatorleistung von 28 g $Ac_2O/g_{Rh} \cdot h$, bei einer Selektivität von 96%.

Beispiel 4

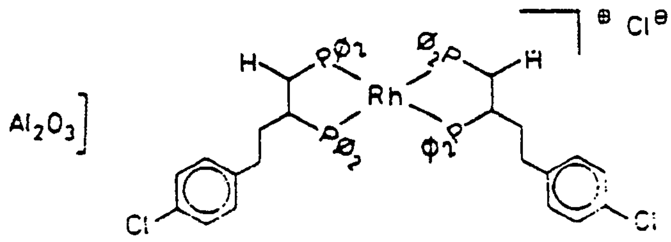

10.0 g aktivierte Aluminiumoxidkugeln (99% $Al_2O_3$) mit einem Durchmesser von 3 mm, einer inneren Oberfläche nach BET von 125 $m^2/g$ und einem Porenvolumen von 0.9 ml/g wurden mit 50 ml einer Lösung aus 117.6 mg des Rhodiumkomplexes 8 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charakterisierung : | Gelbe Kugeln |
| Rh-Gehalt : | 0.1 Gew.% |

2 ml (1.86 g) Essigsäuremethylester, 0.5 ml (1.14 g) Methyljodid und 5.04 g des Katalysators werden im Autoklaven mit Kohlenmonoxid bei 15 bar CO-Druck und 180°C umgesetzt. Nach 1 h Reaktionszeit ergibt sich eine Katalysatorleistung von 44 g $Ac_2O/g_{Rh} \cdot h$, bei einer Selektivität von 97%.

## Ansprüche

1. Trägerkatalysator für die Herstellung von Monocarbonsäureanhydriden durch Carbonylierung der entsprechenden Ester oder Ether, worin das Trägermaterial eine Edelmetall-Chelatverbindung trägt, die aus einer Edelmetallverbindung der 8. Nebengruppe des Periodensystems der Elemente und einem Chelator mit Organostickstoff-, Organophosphor-, Organoarsen-, Organoschwefel- oder Mercaptogruppen gebildet ist, dadurch gekennzeichnet, daß der Chelator in seinem Grundkörper mit Alkyl-, Aryl- oder Aralkylgruppen substituiert ist und eine der folgenden Strukturformeln aufweist :

a)

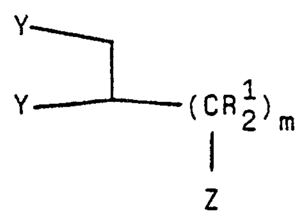

b)

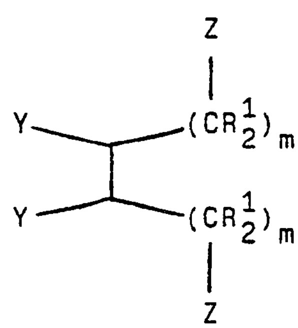

wobei

Y = $-NR^2_2$, ein stickstoffhaltiger Arylrest, $-AsR^2_2$, $-PR^2_2$, $-SR^2$ oder $-SH$ ;
Z = $-H$, Aryl, Phenyl (ggf. ortho-, meta- oder parasubstituiert)
$R^1$ = $-H$, $C_1$ bis $C_3$-Alkyl ;
$R^2$ = $C_1$ bis $C_6$-Alkyl $C_5$ oder $C_6$-Cycloalkyl, $-C_6H_5$ oder $C_6H_5\,CH_2-$, die ggf. mit Halogen-, Methoxy-, Ethoxy- oder $C_1$ bis $C_3$-Alkylgruppen substituiert sind ;
m = 2-6, vorzugsweise 2-4.

2. Trägerkatalysator nach Anspruch 1, dadurch gekennzeichnet, daß in ihm das Trägermaterial zusätzlich eine Nichtedelmetall-Chelatverbindung trägt, die aus einer Nichtedelmetallverbindung aus der 6. oder 8. Nebengruppe des Periodensystems der Elemente und einem Chelator nach Anspruch 1 gebildet ist.

3.Trägerkatalysator nach Anspruch 1 oder 2, dadurch gekennzeichnet, daß er zusätzlich Nichtedelmetallverbindungen aus der 1. bis 3. Hauptgruppe oder der 4. bis 6. oder der 8. Nebengruppe des Periodensystems der Elemente als Promotoren enthält.

4. Trägerkatalysator nach einem der Ansprüche 1-3, dadurch gekennzeichnet, daß er ein anorganisches oxidisches Trägermaterial oder einen Aktivkohleträger enthält.

5. Trägerkatalystor nach einem der Ansprüche 1-4, dadurch gekennzeichnet, daß er zusammen 0,01 bis 50 Gew%, vorzugsweise 0,1 bis 20 Gew%, Chelatverbindungen und ggf. Nichtedelmetallverbindungen enthält.

6. Trägerkatalysator nach einem der Ansprüche 1 bis 5, gekennzeichnet durch die Formel

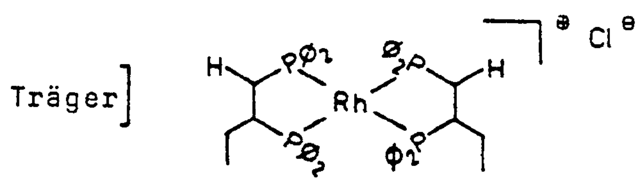

7. Trägerkatalysator nach einem der Ansprüche 1 bis 5, gekennzeichnet durch die Formel

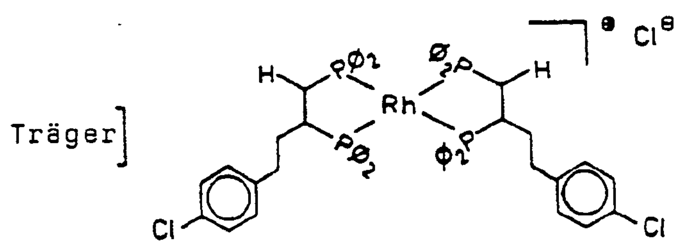

8. Trägerkatalysator nach einem der Ansprüche 1 bis 5, gekennzeichnet durch die Formel

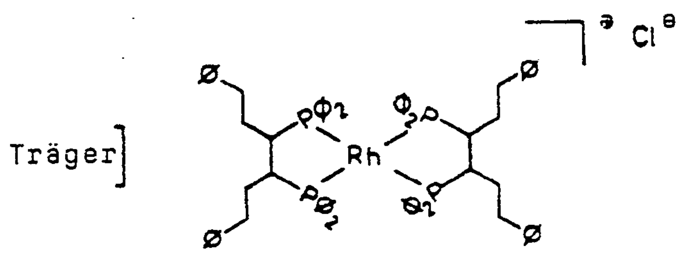

## Revendications

1. Catalyseur sur support pour la fabrication d'anhydrides d'acides monocarboxyliques par cabonylation des esters ou éthers correspondants, dans lequel le matériau de support porte un composé chélate de métal noble, qui est constitué d'un composé de métal noble du 8$^e$ sous-groupe de la classification périodique des éléments et d'un chélatant à groupes organoazotés, organophosphorés, organoarséniés, organosoufrés ou mercapto, caractérisé en ce que le chélatant est substitué dans son corps de base par des groupes alkyles, aryles ou arylalkyles et présente l'une des formules de structure suivantes :

b) 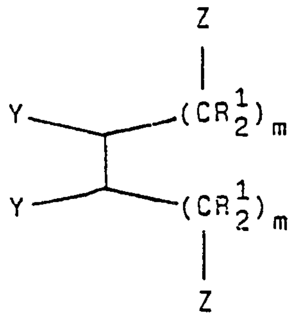

a) 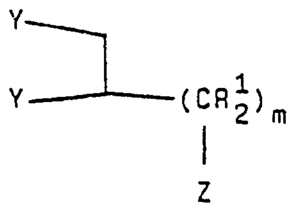

dans lesquelles Y est un groupe $-NR^2_2$, un reste aryle azoté, un groupe $-AsR^2_2$, $-PR^2_2$, $-SR^2$ ou $-SH$ ;
Z est un atome d'hydrogène ou un groupe aryle, phényle (éventuellement ortho-, méta- ou para-substitué) ;
$R^1$ est un atome d'hydrogène ou un groupe alkyle en $C_1$-$C_3$,
$R_2$ est un groupe alkyle en $C_1$-$C_6$, cycloalkyle en $C_5$ ou $C_6$, $C_6H_5$ ou $C_6H_5CH_2$, qui sont éventuellement substitués par des atomes d'halogène ou des groupes méthoxy, éthoxy ou alkyles en $C_1$-$C_3$ ;
m est un nombre de 2 à 6, de préférence de 2 à 4.

2. Catalyseur sur support selon la revendication 1, caractérisé en ce que le matériau de support porte en outre un composé chélate de métal non noble, qui est constitué d'un composé de métal non noble du 6e ou 8e sous-groupe de la classification périodique des éléments et d'un chélatant selon la revendication 1.

3. Catalyseur sur support selon la revendication 1 ou 2, caractérisé en ce qu'il contient en outre des composés de métaux non nobles des 1er à 3e groupes principaux ou du 4e au 6e ou du 8e sous-groupe de la classification périodique des éléments comme promoteurs.

4. Catalyseur sur support selon l'une des revendications 1 à 3, caractérisé en ce qu'il contient un matériau de support inorganique du type oxyde ou un support de charbon actif.

5. Catalyseur sur support selon l'une des revendications 1 à 4, caractérisé en ce qu'il contient à la fois 0,01 à 50% en poids, de préférence 0,1 à 20% en poids, de composés chélates et éventuellement des composés de métaux non nobles.

6. Catalyseur sur support selon l'une des revendications 1 à 5, caractérisé par la formule

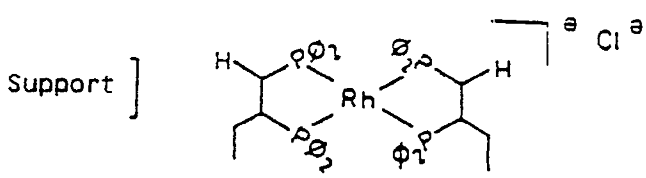

7. Catalyseur sur support selon l'une des revendications 1 à 5, caractérisé par la formule

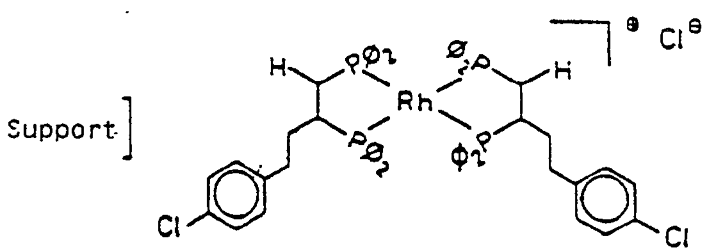

8. Catalyseur sur support selon l'une des revendications 1 à 5, caractérisé par la formule

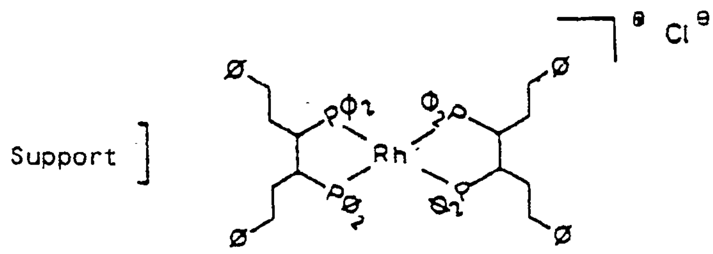

## Claims

1. A supported catalyst for the preparation of monocarboxylic anhydrides by carbonylation of the corresponding esters or ethers, in which the support material supports a noble metal chelate compound which is formed by a noble metal compound from subgroup VIII of the periodic table of the elements and a chelating agent containing organonitrogen, organophosphorus, organoarsenic, organosulfur or mercapto groups, wherein the chelating agent is substituted in its basic structure by alkyl, aryl or aralkyl groups and has one of the following structural formulae :

a)

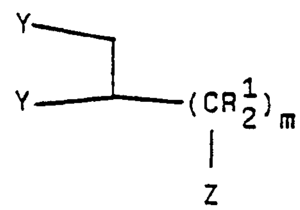

b)

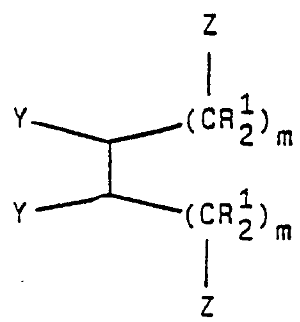

in which

Y is $-NR^2_2$, a nitrogen-containing aryl radical, $-AsR^2_2$, $-PR^2_2$, $-SR^2$ or $-SH$ ;

Z is −H, aryl, phenyl (if desired, ortho-, meta- or para-substituted) ;

$R^1$ is −H, $C_1$ to $C_3$-alkyl ;

$R^2$ is $C_1$– to $C_6$-alkyl, $C_5$ or $C_6$-cycloalkyl, $-C_6H_5$ or $C_6H_5CH_2-$, which may be substituted by halogen, methoxy, ethoxy or $C_1$– to $C_3$– alkyl groups

m is 2-6, preferably 2-4.

2. The supported catalyst as claimed in claim 1, wherein the support material in the supported catalyst additionally supports a base metal chelate compound which is formed from a base metal compound from subgroup 6 or 8 of the periodic table of the elements and a chelating agent as claimed in claim 1.

3. The supported catalyst as claimed in claim 1 or 2, wherein the supported catalyst additionally contains

base metal compounds from main groups 1 to 3 or subgroups 4 to 6 or 8 of the periodic table of the elements as promoters.

4. The supported catalyst as claimed in any of claims 1 to 3, wherein the supported catalyst contains an inorganic oxidic support material or an activated carbon support.

5. The supported catalyst as claimed in any of claims 1 to 4, wherein the supported catalyst altogether contains 0.01 to 50% by weight, preferably 0.1 to 20% by weight, of chelate compounds and optionally base metal compounds.

6. The supported catalyst as claimed in any of claims 1 to 5, having the formula

Support

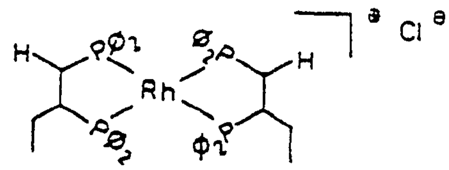

7. The supported catalyst as claimed in any of claims 1 to 5, having the formula

Support

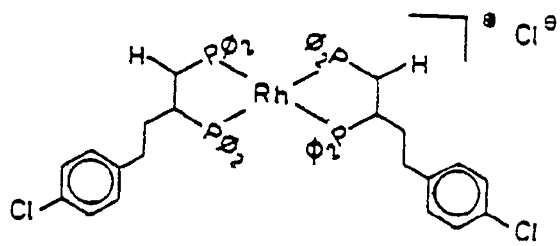

8. The supported catalyst as claimed in any of claims 1 to 5, having the formula

Support